(19) Europäisches Patentamt
European Patent Office
Office européen des brevets

(11) Veröffentlichungsnummer: **0 004 959**
**A1**

(12) # EUROPÄISCHE PATENTANMELDUNG

(21) Anmeldenummer: **79101159.6**

(22) Anmeldetag: **17.04.79**

(51) Int. Cl.[2]: **C 07 C 127/15**
C 07 C 127/19, C 07 C 129/12
C 07 C 157/05, C 07 C 157/09
C 07 C 157/14, A 61 K 31/17
A 61 K 31/155, A 61 K 31/275

(30) Priorität: **24.04.78 LU 79504**

(43) Veröffentlichungstag der Anmeldung:
**31.10.79 Patentblatt 79/22**

(84) Benannte Vertragsstaaten:
**BE CH DE FR GB IT LU NL SE**

(71) Anmelder: **CIBA-GEIGY AG**
**Patentabteilung Postfach**
**CH-4002 Basel(CH)**

(72) Erfinder: **Kühnis, Hans, Dr.**
**Lange Gasse 26**
**CH-4052 Basel(CH)**

(72) Erfinder: **Egli, Christian, Dr.**
**Auf Egg**
**CH-4465 Magden(CH)**

(72) Erfinder: **Eichenberger, Kurt, Dr.**
**Neubergweg 36**
**CH-4106 Therwil(CH)**

(54) **Verfahren zur Herstellung von 3-Oxo-cyclopentenylharnstoffen, die so erhaltenen Verbindungen, sie enthaltende pharmazeutische Präparate und 3-Oxo-cyclopentenylisothioharnstoffe.**

(57) 3-Oxo-cyclopentenylharnstoffe der Formel

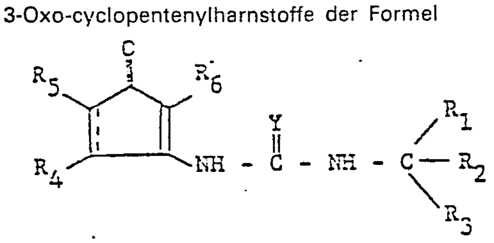

(I)

deren Cyclopentenylring eine weitere Doppelbindung aufweisen kann und worin $R_1$ Wasserstoff oder Nideralkyl, $R_2$ Niederalkyl und $R_3$ gegebenenfalls ungesättigtes Niederalkyl, oder $R_2$ und $R_3$ zusammen mit dem sie verbindenden C-Atom gegebenenfalls ungesättigtes Cycloalkyl oder $R_1$, $R_2$ und $R_3$ zusammen mit dem sie verbindenden C-Atom gegebenenfalls substituiertes Phenyl, $R_4$ Wasserstoff, Niederalkyl oder gegebenenfalls substituiertes Phenyl, $R_5$ Wasserstoff, eine gegebenenfalls veresterte oder amidierte Carboxy- oder Nitrilgruppe und $R_6$ Wasserstoff oder Niederalkyl darstellen und Y für Sauerstoff, Schwefel, die Iminogruppe = NH oder die Cyanoiminogruppe = N-CN steht, Verfahren zu ihrer Herstellung, pharmazeutische Präparate enthaltend diese Verbindungen und ihre Verwendung vorzugsweise in Form von pharmazeutischen Präparaten als Antihypertensiva.

EP 0 044 959 A1

0004959

BEZEICHNUNG GEÄNDERT
siehe Titelseite

CIBA-GEIGY AG
Basel (Schweiz)

4-11680/+

## Verfahren zur Herstellung neuer Harnstoffe

Die Erfindung betrifft neue 3-Oxo-cyclopentenylharnstoffe der Formel

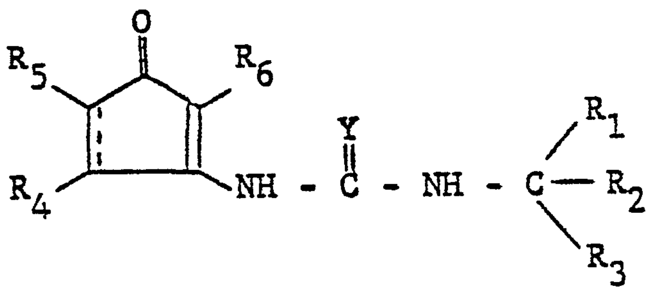

(I)

deren Cyclopentenylring eine weitere Doppelbindung aufweisen kann und worin $R_1$ Wasserstoff oder Niederalkyl, $R_2$ Niederalkyl und $R_3$ gegebenenfalls ungesättigtes Niederalkyl, oder $R_2$ und $R_3$ zusammen mit dem sie verbindenden C-Atom gegebenenfalls ungesättigtes Cycloalkyl oder $R_1$, $R_2$ und $R_3$ zusammen mit dem sie verbindenden C-Atom gegebenenfalls substituiertes Phenyl, $R_4$ Wasserstoff, Niederalkyl oder gegebenenfalls substituiertes Phenyl, $R_5$ Wasserstoff, eine gegebenenfalls veresterte oder amidierte Carboxy- oder Nitrilgruppe und $R_6$ Wasserstoff oder Niederalkyl darstellen und Y für Sauerstoff, Schwefel, die Iminogruppe =NH oder die Cyanoiminogruppe =N-CN steht, sowie Verfahren zu ihrer Herstellung, ferner pharmazeutische Präparate enthaltend diese Verbindungen und ihre Verwendung vorzugsweise in Form von pharmazeutischen Präparaten.

Die im Zusammenhang mit der vorliegenden Beschreibung mit "nieder" bezeichneten Reste und Verbindungen enthalten vorzugsweise bis 7 und in erster Linie bis 4 Kohlenstoffatome.

Ein gegebenenfalls ungesättigtes Niederalkyl ist vorzugsweise ein Niederalkyl, aber auch Niederalkenyl oder Niederalkinyl.

Ein gegebenenfalls ungesättigtes Cycloalkyl ist vorzugsweise ein Cycloalkyl mit 5-7 Ringkohlenstoffatomen, z.B. Cyclopentyl, Cyclohexyl oder Cycloheptyl, aber auch ein Cycloalkenyl mit 5-7 Ringkohlenstoffatomen, beispielsweise Cyclopentenyl, Cyclohexenyl oder Cycloheptenyl, dessen Doppelbindung sich in erster Linie in 1-Stellung befindet.

Ein substituiertes Phenyl ist z.B. einfach, zweifach oder auch mehrfach substituiertes Phenyl. Substituenten sind u.a. Niederalkyl, Niederalkoxy, Halogen, Trifluormethyl, Niederalkylsulfonyl oder Nitro.

Gegebenenfalls verestertes oder amidiertes Carboxy ist die Carboxygruppe selbst, ferner z.B. Niederalkoxycarbonyl wie Methoxycarbonyl oder Phenylniederalkoxy-carbonyl oder Carbamoyl, Mono- oder Diniederalkylcarbamoyl, in erster Linie Methyl- oder Dimethyl-carbamoyl.

Niederalkyl ist z.B. n-Propyl, Isopropyl, n-Butyl, Isobutyl oder tert.-Butyl, und in erster Linie Methyl oder Aethyl.

Niederalkenyl ist z.B. Vinyl, Allyl, 2- oder 3-Methallyl oder 3,3-Dimethylallyl.

Niederalkinyl ist insbesondere Propargyl.

Niederalkoxy ist z.B. n-Propyloxy, n-Butyloxy, Isopropyloxy oder Isobutyloxy und in erster Linie Methoxy oder Aethoxy.

Niederalkylsulfonyl ist insbesondere Methylsulfonyl, Aethylsulfonyl oder Propylsulfonyl.

Halogen ist vorzugsweise Halogen mit Atomnummer von 9 bis zu 35, d.h. Fluor, Chlor oder Brom.

Die neuen Verbindungen besitzen wertvolle pharmakologische Eigenschaften.

Die Erfindung betrifft insbesondere Verbindungen der Formel II

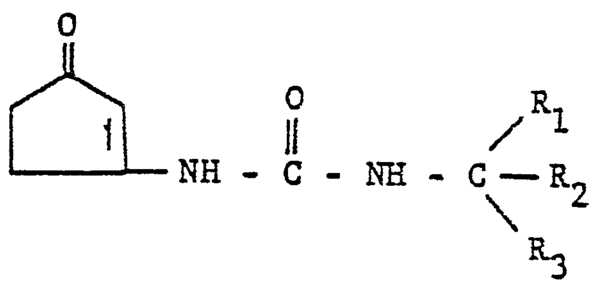

(II)

worin $R_1$, $R_2$ und $R_3$ Niederalkyl oder worin $R_1$ Niederalkyl und $R_2$ und $R_3$ zusammen mit dem sie verbindenden C-Atom Cycloalkyl mit 5-7 Kohlenstoffatomen bedeuten.

Die Erfindung betrifft vor allem Verbindungen der Formel II, worin $R_1$ und $R_2$ Methyl und $R_3$ Niederalkyl mit 1-3 Kohlenstoffatomen oder worin $R_1$ Methyl und $R_2$ und $R_3$ zusammen mit dem sie verbindenden C-Atom Cycloalkyl mit 5-7, insbesondere mit 5 Kohlenstoffatomen bedeuten.

In erster Linie betrifft die Erfindung Verbindungen der Formel (II), worin $R_1$, $R_2$ und $R_3$ Methyl bedeuten.

Insbesondere betrifft die Erfindung die neuen, in den Beispielen beschriebenen Verbindungen.

Die neuen Verbindungen der Formel I können nach an sich bekannten Methoden erhalten werden.

So kann man sie herstellen, wenn man eine Verbindung der Formel III

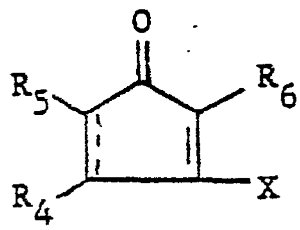

(III)

worin X gegebenenfalls verestertes oder veräthertes Hydroxy ist, mit einer Verbindung der Formel IV

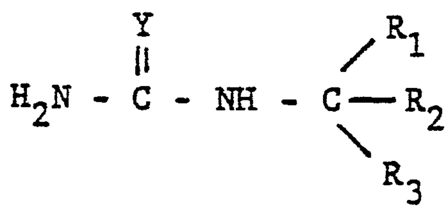

(IV)

umsetzt.

Ein gegebenenfalls verestertes oder veräthertes Hydroxy ist vor allem Hydroxy, das unter Umlagerung der Doppelbindung in 2,3-Stellung des Cyclopentenylrestes auch als Oxo vorliegen kann. Verestertes Hydroxy ist in erster Linie ein mit starken anorganischen oder organischen Säuren, z.B. Halogenwasserstoff-oder Schwefelsäure, oder gegebenenfalls durch Niederalkyl, Niederalkoxy oder Halogen substituierte Benzolsulfonsäure wie p-Toluolsulfonsäure oder Niederalkansulfonsäure, wie Methansulfonsäure, verestertes Hydroxy. Veräthertes Hydroxy ist insbesondere Niederalkoxy, wie Methoxy oder Aethoxy.

Die Umsetzung wird vorzugsweise unter wasser- bzw. säureabspaltenden Bedingungen durchgeführt. So setzt man eine Verbindung III

worin X Hydroxy bedeutet, vorzugsweise in Gegenwart einer starken Säure, wie einer Sulfonsäure z.B. p-Toluolsulfonsäure oder Methansulfonsäure um. Dabei arbeitet man vorzugsweise in einem aprotischen Lösungsmittel, wie einem aromatischen Kohlenwasserstoff, z.B. Benzol, Toluol oder Xylol. Man kann diese Umsetzung jedoch auch so vornehmen, dass man während der Reaktion z.B. durch Destillation das entstehende Wasser laufend entfernt. Die Umsetzung mit Verbindungen der Formel III, worin X verestertes oder veräthertes Hydroxy darstellt, wird vorzugsweise in einem Lösungsmittel, wie einem Alkanol, z.B. Methanol, Aethanol oder Propanol, Diniederalkylformamid, z.B. Dimethylformamid oder Hexamethylphosphorsäuretriamid, wenn erwünscht, in Gegenwart einer Base, z.B. Alkali- oder Erdalkalialkoholat oder -hydrid, einer Stickstoffbase, wie Triniederalkylamin, z.B. Trimethylamin, sowie Pyridin oder Piperidin vorgenommen.Arbeitet man in einem Alkohol als Lösungsmittel verwendet man als Base in erster Linie das entsprechende Natrium- oder Kalium-alkoholat, wird in Dimethylformamid gearbeitet, nimmt man als Base in erster Linie ein Alkali- oder Erdalkalihydrid, z.B. Natrium- oder Kaliumhydrid.

Die zu diesem Verfahren zu verwendenden Ausgangsstoffe sind bekannt oder lassen sich auf an sich bekannte Weise erhalten, z.B. durch Veresterung oder Verätherung der Verbindung der Formel III, worin X Hydroxy ist.

Die neuen Verbindungen werden auch erhalten, wenn man eine Verbindung der Formel

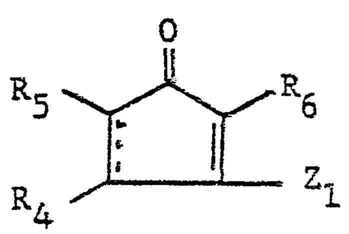

(V)

mit einer Verbindung der Formel VI

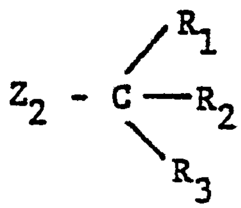

(VI)

umsetzt, worin einer der Reste $Z_1$ und $Z_2$ einer Aminogruppe und der andere der Gruppe der Formel VII

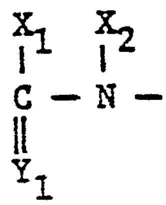

(VII)

entspricht, worin $X_1$ gegebenenfalls reaktionsfähig verestertes oder veräthertes Hydroxy oder Mercapto und $X_2$ Wasserstoff bedeutet, oder $X_1$ und $X_2$ zusammen eine Bindung zwischen C und N darstellen, und $Y_1$ Sauerstoff, Schwefel, Imino oder Cyanimino bedeutet, mit der Massgabe, dass falls $Y_1$ Imino oder Cyanimino bedeutet, $X_1$ Halogen und $X_2$ Wasserstoff darstellen.

Eine reaktionsfähige veresterte Hydroxy- oder Mercaptogruppe $X_1$ ist insbesondere eine durch eine starke anorganische Säure, insbesondere Halogenwasserstoffsäure, z.B. Brom- oder Chlorwasserstoffsäure veresterte Hydroxygruppe. Eine reaktionsfähig verätherte Hydroxy- oder Mercaptogruppe ist in erster Linie Niederalkoxy, wie Methoxy oder Aethoxy oder Niederalkylmercapto, wie Methylmercapto.

Diese Umsetzung wird vorzugsweise in einem inerten organischen Lösungsmittel, wie Benzol, Dioxan, Toluol oder Xylol bei Raum- oder mässig erhöhter Temperatur vorgenommen.

Die Ausgangsstoffe sind bekannt oder lassen sich in an sich bekannter Weise herstellen, so kann man das Cyclopentenyl z.B. durch Curtius-Abbau aus der 3-Oxo-1-cyclopentencarbonsäure erhalten.

In erhaltenen Verbindungen, worin Y die Iminogruppe oder das

Schwefelatom bedeutet, kann man diese in das Sauerstoffatom überführen, z.B. mittels Hydrolyse, vorzugsweise in Gegenwart eines sauren Mittels wie einer Mineralsäure, z.B. Chlorwasserstoffsäure. Man kann aber auch Verbindungen, worin Y Schwefel bedeutet, z.B. durch Umsetzung mit einem Niederalkylhalogenid in die entsprechende Niederalkylmercaptoverbindungen überführen, und diese, z.B. mit Ammoniak oder Cyanamid umsetzen. Dabei arbeitet man in Ab- oder vorzugsweise in Anwesenheit eines Lösungs- oder Verdünnungsmittels und, wenn notwendig, unter Kühlen oder Erwärmen z.B. in einem Temperaturbereich von etwa 0°C bis 150°C, in einem geschlossenen Gefäss und/oder in einer Inertgasatmosphäre.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den eingangs als besonders bevorzugt aufgeführten Verbindungen gelangt.

Die neuen Verbindungen zeigen eine blutdrucksenkende Wirkung, wie sich im Tierversuch, z.B. bei i.v. Gabe in Dosen von 1 bis 30 mg/kg an der narkotisierten Katze zeigen lässt, sowie eine antihypertensive Wirkung wie sich an der renalhypertonischen Ratte in Dosen von etwa 10 bis etwa 100 mg/kg/Tag p.o. zeigen lässt. Sie sind weniger toxisch als vergleichbare, bekannte Verbindungen. Die neuen Verbindungen können daher als blutdrucksenkende Mittel und als Antihypertensiva verwendet werden.

Die neuen Verbindungen der vorliegenden Erfindung können, z.B. zur Herstellung von pharmazeutischen Präparaten verwendet werden, welche eine wirksame Menge der Aktivsubstanz zusammen oder im Gemisch

mit anorganischen oder organische, festen oder flüssigen, pharmazeutisch verwendbaren Trägerstoffen enthalten, die sich zur enteralen oder parenteralen Verabreichung eignen. So verwendet man Tabletten oder Gelatinekapseln, welche den Wirkstoff zusammen mit Verdünnungsmitteln, z.B. Laktose, Dextrose, Sukrose, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidin, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel. Ferner kann man die neuen pharmakologisch wirksamen Verbindungen in Form von injizierbaren, z.B. intravenös verabreichbaren Präparaten oder von Infusionslösungen verwenden. Solche Lösungen sind vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch aktive Stoffe enthalten können, werden in an sich bekannter Weise, z.B. mittels konventioneller Misch-, Granulier-, Dragier-, Lösungs- oder Lyophilisierungsverfahren, hergestellt und enthalten von etwa 0,1 % bis 100 %, insbesondere von etwa 1 % bis etwa 50 %, Lyophilisate bis zu 100 % des Aktivstoffes.

Die Dosierung kann von verschiedenen Faktoren, wie Applikationsweise, Spezies, Alter und/oder individuellem Zustand abhängen. Die täglich zu verabreichende Dosis liegen bei oraler Applikation zwischen etwa 10 mg und etwa 1000 mg für Warmblüter mit einem Gewicht

von etwa 70 kg.

Die Erfindung betrifft auch die als Zwischenprodukt erhältlichen Isothioharnstoffe der Formel VIII

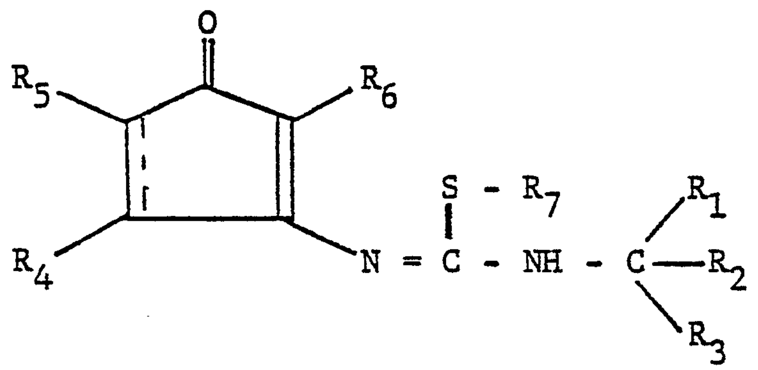

(VIII)

worin $R_1$ bis $R_6$ die obengenannte Bedeutung haben und $R_7$ für Niederalkyl, besonders Methyl steht.

Die folgenden Beispiele dienen zur Illustration der Erfindung: Temperaturen werden in Celsiusgraden angegeben.

Beispiel 1: 9,8 g 1,3-Cyclopentadion, 11,6 g t-Butylharnstoff, 0,2 g p-Toluolsulfonsäure werden in 300 ml Benzol-Toluol (1:1) 6 Stunden am Wasserabscheider erhitzt. Die organische Phase wird abgetrennt, der Rückstand in 200 ml heissem Essigester suspendiert und von unlöslichem Harz abfiltriert. Das Filtrat wird eingeengt, mit Aether versetzt und abgekühlt. Das ausgefallene Kristallisat wird abfiltriert und mit wenig Essigester nachgewaschen. Das Filtrat wird mit der oben abgetrennten organischen Phase vereinigt, eingedampft und an Silicagel in einem Gemisch von Essigester-Methanol-Ammoniak (70:30:1) chromatographiert. Durch anschliessende Sublimation wird noch vorhandener t-Butylharnstoff abgetrennt. Der 1-tert.-Butyl-3-(2-cyclopenten-1-on-3-yl)-harnstoff hat einen Smp. 193-195°.

Auf gleiche Weise kann der 1-tert.-Amyl-3-(2-cyclopenten-1-on-3-yl)-harnstoff, Smp. 208-209°, hergestellt werden.

Beispiel 2: 0,5 g 1-Amino-cyclopenten-(1)-on-(3), 5 ml t-Butylisocyanat und 5 ml Dimethylformamid werden in einem Bombenrohr 18 Stunden auf 140° erhitzt. Es wird abgekühlt, Methanol zugegeben, filtriert und das Filtrat zur Trockne eingedampft. Der Rückstand wird mit Essigester ausgekocht, von einem kohleartigen Produkt abfiltriert und das Filtrat eingedampft und an Kieselgel in Essigester-Methanol (8:2) chromatographiert. Der 1-tert.-Butyl-3-(2-cyclopenten-1-on-3-yl)-harnstoff wird aus Isopropanol-Aether umkristallisiert. Er besitzt einen Smp. von 193-195°.

Beispiel 3: Zu einer aus 4,6 g Natrium und 300 ml absolutem Aethanol hergestellten Natriumalkoholatlösung werden 18,4 g t-Butyl-harnstoff unter Rühren zugegeben und 15 Minuten auf 60° erhitzt. Anschliessend werden 19,9 g 1-Aethoxy-cyclopenten-(1)-on-(3) in 25 ml absolutem Aethanol während 5 Minuten zugetropft und die klare Lösung 10 Stunden unter Rückfluss erhitzt. Dann wird zur Trockne eingedampft, der Rückstand in 100 ml Wasser gelöst und 5 mal mit je 150 ml Essigester ausgezogen. Die organischen Phasen werden vereinigt, getrocknet und eingedampft. Der Rückstand wird aus Isopropanol-Aether umkristallisiert. Der 1-tert.-Butyl-3-(2-cyclopenten-1-on-3-yl)-harnstoff hat einen Smp. von 193-195°.

Auf gleiche Weise kann der 1-tert.-Butyl-3-(2-methyl-2-cyclopenten-1-on-3-yl)-harnstoff, Smp. 216-220° und der 1-(1-Methyl-1-cyclopentyl)-3-(2-cyclopenten-1-on-3-yl)-harnstoff, Smp. 182-183° hergestellt werden.

Beispiel 4: 7,5 g 1-Amino-cyclopenten-(1)-on-(3) werden in 150 ml absolutem Dimethylformamid mit 8,6 g Kalium-tert.-butylat versetzt und unter Stickstoff und Rühren 1 Stunde auf 90° erwärmt. Die hellbraune, dicke Suspension wird abgekühlt und unter Eiskühlung 11,1 g tert.-Butylthiocyanat in 35 ml absolutem Dimethylformamid zugetropft. Die entstandene Lösung wird 15 Stunden bei Zimmertemperatur ausgerührt. Dann wird mit 77 ml 1-n Salzsäure neutralisiert, zur Trockne eingedampft und die anhaftende Feuchtigkeit mit Toluol abgetrieben. Den Rückstand suspendiert man in 250 ml warmen Methanol, filtriert ab und wäscht mit Methanol und Aether. Das Methanolfiltrat wird zur Trockne eingedampft, der Rückstand mit 250 ml Aether gekocht und abfiltriert. Das Kristallisat wird dann an Kieselgel in Essigester-Methanol 8:2 chromatographiert. Hierauf wird in Methanol mit Tierkohle behandelt, das Filtrat zur Trockne eingedampft und mit Toluol getrocknet. Der Rückstand wird aus Isopropanol umkristallisiert. Der 1-tert.-Butyl-3-yl)-thioharnstoff hat einen Smp. von 198-200°

Beispiel 5: Ein Gemisch von 2,26 g 1-tert.-Butyl-3-(2-cyclopenten-1-on-3-yl)-S-methyl-thioharnstoff, 0,6 g fein pulverisiertem Cyanamid und 10 ml absolutem Dimethylformamid, wird unter Zusatz von 3 Tropfen N,N-Diisopropyl-äthylamin 48 Stunden bei Raumtemperatur gerührt. Danach wird unter vermindertem Druck eingedampft. Den Rückstand chromatographiert man an 100 Kieselgel, wobei ein Gemisch von Chloroform-Methanol (9:1) als Eluiergemisch verwendet wird. Nach Umkristallisation des gereinigten Produktes aus Essigsäureäthylester-Petroläther erhält man das 1-tert.-Butyl-2-cyano-3-(2-cyclopenten-1-on-3-yl)-guanidin vom F. = 211 - 214°.

Der als Ausgangsstoff verwendete 1-tert.-Butyl-3-(2-cyclopenten-1-on-3-yl)-S-methyl-thioharnstoff kann auf folgende Weise hergestellt werden:

Zu einem Gemisch von 1,05 g 1-tert.-Butyl-3-(2-cyclopenten-1-on-3-yl)-thioharnstoff, 0,7 g Kaliumcarbonat in 25 ml absolutem Aethylalkohol, tropft man unter Rühren eine Lösung von 0,37 ml Methyljodid in 5 ml absolutem Aethylalkohol. Man lässt noch 20 Stunden bei Raumtemperatur ausrühren, und dampft dann unter vermindertem Druck ein. Den Rückstand suspendiert man in 50 ml Chloroform und filtriert über Hyflo. Das Filtrat dampft man unter vermindertem Druck ein, und befreit den Rückstand durch Behandeln mit Toluol von noch anhaftender Feuchtigkeit. Nach Anreiben des Rückstandes mit Diäthyläther erhält man als kristallines Produkt den 1-tert.-Butyl-3-(2-cyclopenten-1-on-3-yl)-S-methyl-thioharnstoff vom F. 80-84°.

Beispiel 6: 7,5 g 1-Amino-cyclopenten-(1)-on-(3) werden in 150 ml absolutem Dimethylformamid mit 8,6 g Kalium-tert.-butylat versetzt und unter Stickstoff und Rühren 1 Stunde auf 90° erwärmt. Die hellbraune, dicke Suspension wird abgekühlt und unter Eiskühlung 9,5 g sek.-Butylisocyanat in 35 ml absolutem Dimethylformamid zugetropft. Die entstandene Lösung wird 15 Stunden bei Zimmertemperatur ausgerührt. Dann wird mit 77 ml 1-n Salzsäure neutralisiert, zur Trockne eingedampft und die anhaftende Feuchtigkeit mit Toluol abgetrieben. Den Rückstand suspendiert man in 250 ml warmen Methanol, filtriert ab und wäscht mit Methanol und Aether. Das Methanolfiltrat wird zur Trockne eingedampft, der Rückstand mit 250 ml Aether gekocht und abfiltriert. Das Kristallisat wird dann an Kieselgel in Essigester-Methanol 8:2 chromatographiert. Hierauf wird in Methanol mit Tierkohle behandelt, das Filtrat zur Trockne eingedampft und mit Toluol getrocknet. Der Rückstand wird aus Isopropanol umkristallisiert. Man erhält so den 1-sek.-Butyl-3-(2-cyclopenten-1-on-3-yl)-harnstoff.

Beispiel 7: 4,7 g Kalium-tert.-butylat werden in 100 ml absolutem Dimethylformamid unter Rühren vorgelegt. Dazu gibt man bei leicht exothermer Reaktion bis 35°C, 4,9 g N''-cyano-N'-tert.-butyl-guanidin und rührt das Gemisch 2 Stunden bei 50°C Reaktionstemperatur. Dann wird innert 30 Minuten eine Lösung von 4,4 g 1-Aethoxy-cyclopentan-(1)-on-(3) in 50 ml absolutem Dimethylformamid zugetropft und noch weitere 3 Stunden bei 50°C gerührt. Dann wird abgekühlt, mit 42 ml 1-n Salzsäure neutralisiert und zur Trockne eingedampft. Den Rückstand löst man in Chloroform und wäscht mit Wasser. Nach dem Trocknen der vereinigten organischen Phasen entfärbt man mit Florisil und dampft ein. Der Rückstand wird aus Isopropylalkohol-Diäthyläther umkristallisiert, und man erhält als weises Kristallisat das 1-tert.-Butyl-2-cyano-3-(2-cyclopenten-1-on-3-yl)-guanidin, vom Smp. 211-214°C.

Auf gleiche Weise kann das tert. Amyl-2-cyano-3-(2-cyclopenten-1-on-3-yl)-guanidin vom Smp. 135.5-137.5°.erhalten werden.

0004959

Patentansprüche

1. Verfahren zur Herstellung neuer 3-Oxo-cyclopentenyl-harnstoffe der Formel I

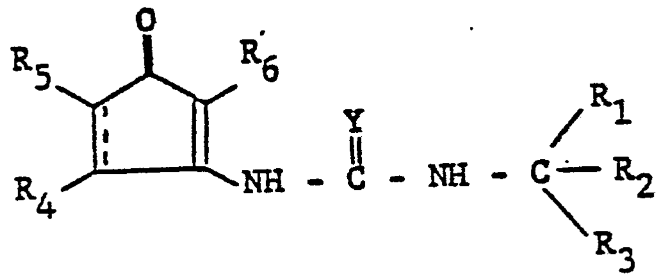

(I)

deren Cyclopentenylring eine weitere Doppelbindung aufweisen kann und worin $R_1$ Wasserstoff oder Niederalkyl, $R_2$ Niederalkyl und $R_3$ gegebenenfalls ungesättigtes Niederalkyl, oder $R_2$ und $R_3$ zusammen mit dem sie verbindenden C-Atom gegebenenfalls ungesättigtes Cycloalkyl oder $R_1$, $R_2$ und $R_3$ zusammen mit dem sie verbindenden C-Atom gegebenenfalls substituiertes Phenyl, $R_4$ Wasserstoff, Niederalkyl oder gegebenenfalls substituiertes Phenyl, $R_5$ Wasserstoff, eine gegebenenfalls veresterte oder amidierte Carboxy- oder Nitrilgruppe und $R_6$ Wasserstoff oder Niederalkyl darstellen und Y für Sauerstoff, Schwefel, die Imino- oder die Cyaniminogruppe steht, dadurch gekennzeichnet, dass man eine Verbindung der Formel III

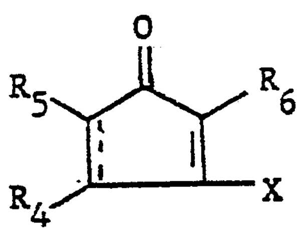

(III)

worin X gegebenenfalls verestertes oder veräthertes Hydroxy ist, mit einer Verbindung der Formel IV

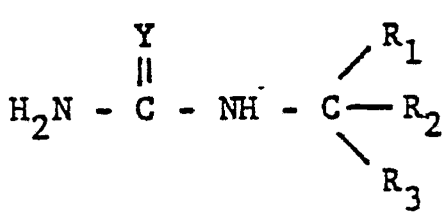

(IV)

umsetzt, oder dass man eine Verbindung der Formel V

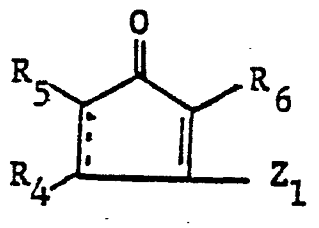

(V)

mit einer Verbindung der Formel VI

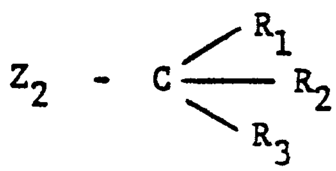

(VI)

umsetzt, worin einer der Reste $Z_1$ und $Z_2$ einer Aminogruppe und der andere der Gruppe VII

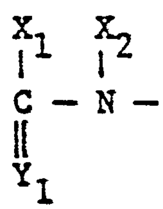

(VII)

entspricht, worin $X_1$ gegebenenfalls reaktionsfähig verestertes oder veräthertes Hydroxy oder Mercapto und $X_2$ Wasserstoff bedeuten, oder $X_1$ und $X_2$ zusammen eine Bindung zwischen C und N darstellen und $Y_1$ Sauerstoff, Schwefel, Imino oder Cyanïmino bedeutet, mit der Massgabe dass, falls $Y_1$ Imino oder Cyanimino bedeutet, $X_1$ Halogen und $X_2$ Wasserstoff darstellen, und, wenn erwünscht, in erhaltenen Verbindungen, worin Y die Iminogruppe oder S bedeutet, diese in das Sauerstoffatom überführt.

2. Verfahren nach Anspruch 1, dadurch gekennzeichnet, dass man von einer auf irgendeiner Stufe des Verfahrens als Zwischenprodukt erhältlichen Verbindung ausgeht und die fehlenden Verfahrensschritte durchführt, oder das Verfahren auf irgendeiner Stufe abbricht.

3. Verfahren nach Anspruch 1 oder 2, dadurch gekennzeichnet, dass man einen Ausgangsstoff unter den Reaktionsbedingungen bildet oder bei denen eine Reaktionskomponente gegebenenfalls in Form ihrer Salze vorliegt.

4. 3-Oxo-cyclopentenyl-harnstoffe der Formel (I)

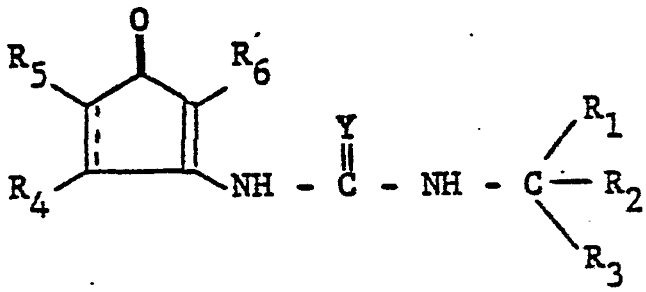

(I)

deren Cyclopentenylring eine weitere Doppelbindung aufweisen kann und worin $R_1$ Wasserstoff oder Niederalkyl, $R_2$ Niederalkyl und $R_3$ gegebenenfalls ungesättigtes Niederalkyl, oder $R_2$ und $R_3$ zusammen mit dem sie verbindenden C-Atom gegebenenfalls ungesättigtes Cycloalkyl oder $R_1$, $R_2$ und $R_3$ zusammen mit dem sie verbindenden C-Atom gegebenenfalls substituiertes Phenyl, $R_4$ Wasserstoff, Niederalkyl oder gegebenenfalls substituiertes Phenyl, $R_5$ Wasserstoff, eine gegebenenfalls veresterte oder amidierte Carboxy- oder Nitrilgruppe und $R_6$ Wasserstoff oder Niederalkyl darstellen und Y für Sauerstoff, Schwefel, die Imino- oder Cyaniminogruppe steht.

5. 3-Oxo-cyclopentenyl-harnstoffe der Formel (I)

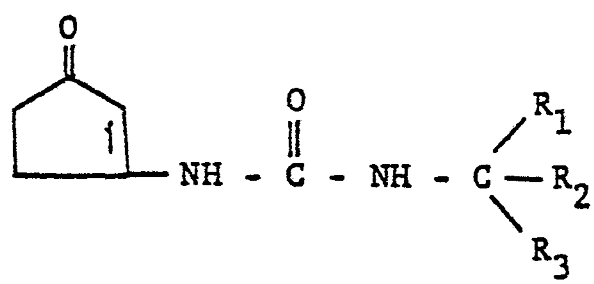

(II)

worin $R_1$, $R_2$ und $R_3$ Niederalkyl oder worin $R_1$ Niederalkyl und $R_2$ und $R_3$ zusammen mit dem sie verbindenden C-Atom Cycloalkyl mit 5-7 Kohlenstoffatomen bedeuten.

6. 3-Oxo-cyclopentenyl-harnstoffe der in Patentanspruch 5 gegebenen Formel II, worin $R_1$ und $R_2$ Methyl und $R_3$ Niederalkyl mit 1-3 Kohlenstoffatomen, oder worin $R_1$ Methyl und $R_2$ und $R_3$ zusammen mit dem sie verbindenden C-Atomen Cycloalkyl mit 5-7 Kohlenstoffatomen bedeutet.

7. 1-tert.-Butyl-3-(2-cyclopenten-1-on-3-yl)-harnstoff.

8. 1-tert.-Amyl-3-(2-cyclopenten-1-on-3-yl)-harnstoff.

9. 1-tert.-Butyl-3-(2-cyclopenten-1-on-3-yl)-thioharnstoff.

10. 1-tert.-Butyl-2-cyano-3-(2-cyclopenten-1-on-3-yl)-guanidin.

11. 1-sek.-Butyl-3-(2-cyclopenten-1-on-3-yl)-harnstoff.

12. 1-tert. Amyl-2-cyano-3-(2-cyclopenten-1-on-3-yl)-guanidin.

13. Pharmazeutische Präparate enthaltend eine der in den Ansprüchen 4-12 beanspruchten Verbindungen.

14. Verwendung einer der in den Ansprüchen 4-12 beanspruchten Verbindungen.

15. Isothioharnstoffe der Formel

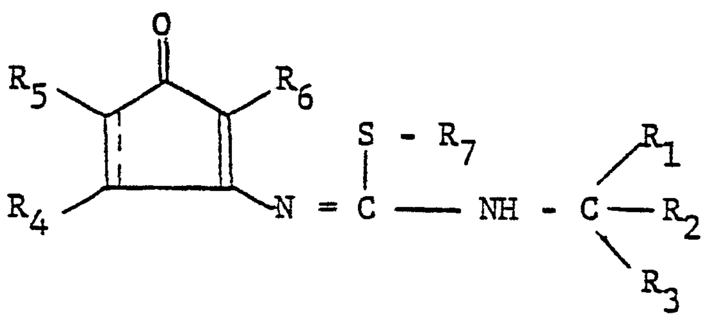

deren Cyclopentenylring eine weitere Doppelbindung aufweisen kann und worin $R_1$ Wasserstoff oder Niederalkyl, $R_2$ Niederalkyl und $R_3$ gegebenenfalls ungesättigtes Niederalkyl, oder $R_2$ und $R_3$ zusammen mit dem sie verbindenden C-Atom gegebenenfalls ungesättigtes Cycloalkyl oder $R_1$, $R_2$ und $R_3$ zusammen mit dem sie verbindenden C-Atom gegebenenfalls

substituiertes Phenyl, $R_4$ Wasserstoff, Niederalkyl oder gegebenenfalls substituiertes Phenyl, $R_5$ Wasserstoff, eine gegebenenfalls veresterte oder amidierte Carboxy- oder Nitrilgruppe und $R_6$ Wasserstoff oder Niederalkyl darstellen.

16. 1-tert.-Butyl-3-(2-cyclopenten-1-on-3-yl)-S-methyl-thioharnstoff.

0004959

Europäisches Patentamt

# EUROPÄISCHER RECHERCHENBERICHT

Nummer der Anmeldung

EP 79 101 159.6

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| X | DE - A - 2 516 556 (E.I. DU PONT DE NEMOURS AND CO.)<br>* Ansprüche 1 und 7 *<br>-- | 4,13 | C 07 C 127/15<br>C 07 C 127/19<br>C 07 C 129/12<br>C 07 C 157/05<br>C 07 C 157/09<br>C 07 C 157/14<br>A 61 K 31/17<br>A 61 K 31/155<br>A 61 K 31/275 |
| A | JOURNAL OF MEDICINAL CHEMISTRY, Band 11, Juli 1968<br>New York<br>M. GADEKAR et al. "Hypotensive Activity of Some Cyanoguanidines"<br>* Seiten 811 bis 814 *<br>-- | | RECHERCHIERTE SACHGEBIETE (Int. Cl.²)<br>A 61 K 31/00<br>C 07 C 127/00<br>C 07 C 129/12<br>C 07 C 157/00 |
| A | JOURNAL OF MEDICINAL CHEMISTRY, Band 11, November 1968<br>New York,<br>J.H. SHORT et al. "Sympathetic Nervous System Blocking Agents. V. Derivatives of Isobutyl-, t-Butyl-, and Neopentylguanidine"<br>* Seiten 1129 bis 1135 *<br>-- | | |
| A | US - A - 3 701 807 (J.P. CHUPP)<br>---- | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| Berlin | 03-08-1979 | STOOS |